# EUROPEAN PATENT APPLICATION

(11) **EP 2 363 123 A1**
(43) Date of publication of application: **07.09.2011**
(21) Application number: 11165690.6
(22) Date of filing: 28.06.2007
(51) Int. Cl.: A61K 31/198, A61P 9/02, A61K 31/275, A61K 31/27, A61K 31/15

(54) **Pharmaceutical compositions comprising droxidopa**

(30) Priority: 28.06.2006 US 806036 P
(62) Divisional of application: 07784556.8
(71) Applicant: Chelsea Therapeutics, Inc., Charlotte, NC 28277 (US)
(72) Inventor: Roberts, Michael, J., CHARLOTTE, NC 28277 (US); Pedder, Simon, FORT MILL, SC 29715 (US)
(74) Representative: Noel, Chantal Odile

(57) **Abstract**

The present invention provides combinations of droxidopa and one or more further pharmaceutically active compounds, said further compounds preferentially being selected from the group of COMT inhibiting compounds, cholinesterase inhibiting compounds, and monoamine oxidase inhibiting compounds. The invention further provides methods of treating conditions, such as orthostatic hypotension, comprising administering the combinations.

## Description

### FIELD OF THE INVENTION

The present invention is directed to combinations of droxidopa with one or more pharmaceutically active compounds. More particularly, the invention is directed to combinations of droxidopa with one or more catechol-O-methyltransferase inhibitors, one or more cholinesterase inhibitors, or one or more monoamine oxidase inhibitors.

### BACKGROUND

Droxidopa is a known synthetic amino acid precursor of norepinephrine that is converted directly to norepinephrine via the action of aromatic L-amino acid decarboxylase, also known as dopa decarboxylase (DDC). Droxidopa is an artificial amino acid that has been used to supplement noradrenaline in neurodegenerative disorders, such as Parkinson's disease; however, multiple pharmacological activities have been observed with droxidopa, including the following: (1) it is directly converted to 1-norepinephrine by the action of the aromatic L-amino acid decarboxylase which is widely distributed in a living body, and thus has an effect of replenishing norepinephrine; (2) it passes through the blood-brain barrier into the brain; (3) it specifically recovers norepinephrine activated nerve functions which have decreased in the central and peripheral nervous system; and (4) it shows various actions via the adrenergic receptors in various tissues. Droxidopa has particularly been indicated as being useful for treatment of peripheral orthostatic hypotension (see U.S. Patent No. 4,330,558, which is incorporated herein by reference in its entirety).

Orthostatic hypotension is not a disease, but is rather a physical finding that is a manifestation of abnormal blood pressure regulation due to various causes. The American Autonomic Society (AAS) and the American Academy of Neurology (AAN) define orthostatic hypotension as a systolic blood pressure decrease of at least 20 mm Hg or a diastolic blood pressure decrease of at least 10 mm Hg within three minutes of standing up from a non-standing position.

While orthostatic hypotension has been observed in all age groups, it occurs more frequently in the elderly population, particularly in frail or sick individuals or those with Parkinson's disease. The condition can be associated with multiple diagnoses, conditions, and symptoms, such as lightheadedness associated with standing, susceptibility to falling, and a history of myocardial infarction or transient ischemic attack. Further, the condition may actually be a predictor of ischemic stroke.

The etiology and pathophysiology of orthostatic hypotension is rather complex, involving multiple body systems and physical responses. The gravitational stress of sudden standing normally causes pooling of blood in the venous capacitance vessels of the legs and trunk (on the order of 300 to 800 mL of blood). Accordingly, maintenance of blood pressure during positional changes, particularly standing, requires coordinated and rapid cardiac, vascular, neurologic, muscular, and neurohumoral responses. Abnormalities in any of these responses can cause a reduction in blood pressure and organ perfusion, particularly cerebral perfusion.

The autonomic nervous system particularly plays an important role in maintaining blood pressure upon positional change. The sympathetic nervous system adjusts the tone in arteries, veins, and the heart. Baroreceptors, which are located primarily in the carotid arteries and aorta, are highly sensitive to changes in blood pressure (such as upon standing from a seated or lying position) and activate autonomic reflexes that rapidly normalize blood pressure by causing a transient tachycardia. These changes reflect primarily the sympathetic mediated increase in catecholamine levels, which augments vasomotor tone of the capacitance vessels, increases heart rate and myocardial contractility, and thereby enhances cardiac output. Arterial and venous vasoconstriction are also mediated by similar mechanisms. Other physiologic mechanisms may also be involved, including low-pressure receptors in the heart and lungs, the renin-angiotensin-aldosterone system, vasopressin, and the systemic release of norepinephrine. Generally, all parts of the cardiovascular and nervous systems must work together to maintain blood pressure upon positional change. If there is inadequate intravascular volume, impairment of the autonomic nervous system, reduction of venous return, or inability of the heart to beat more rapidly or with greater power, orthostatic hypotension may result.

Orthostatic hypotension can be classified as neurogenic, non-neurogenic, or iatrogenic (*e.g*., caused by medication). Neurogenic etiologies include the following: spinal cord problems (such as related to Shy-Drager syndrome, syringomyelia, tabes dorsalis, transverse myelitis, or tumors); peripheral nervous system problems (such as related to HIV/AIDS, alcoholic polyneuropathy, amyloidosis, diabetes mellitus, dopamine beta-hydroxylase deficiency, Guillain-Barré syndrome, paraneoplastic syndrome, renal failure, or vitamin B₁₂ deficiency); and other neurogenic etiologies (such as related to brain-stem lesions, brain tumors, carotid sinus hypersensitivity, cerebral vascular accidents, dysautonomias, multiple sclerosis, neurocardiogenic syncope, Parkinson's disease, pure autonomic failure, or Sepsis Syringobulbia). Non-neurogenic etiologies include the following: cardiac pump failure (such as related to aortic stenosis, bradyarrhythmia, myocardial infarction, myocarditis, pericarditis, or tachyarrhythmia); reduced intravascular volume (such as related to adrenal insufficiency, bums, dehydration, diabetes insipidus, diarrhea, hemorrhage, salt-losing neuropathy, straining with heavy lifting, urination, or defecation, or vomiting); and venous pooling (such as related to alcohol consumption, fever, heat, postprandial dilation of splanchnic vessel beds, prolonged recumbency or standing, sepsis, or vigorous exercise with dilation of skeletal vessel beds). Drugs known to cause orthostatic hypotension include: alpha and beta blockers; antihypertensives; bromocriptine (PARLODEL^{®}); dopamine agonists; diuretics; insulin; MAO inhibitors; marijuana; minor tranquilizers; narcotics/sedatives; nitrates; phenothiazines; sildenafil (VIAGRA^{®}); sympatholytics; sympathomimetics (with prolonged use); tricyclic antidepressants; vasodilators; and vincristine (Oncovin).

Given the broad array of underlying causes, treatment for orthostatic hypotension can vary. The first steps in treatment of orthostatic hypotension are diagnosis and management of the underlying cause. When the causative disease cannot be identified or improved, management is typically designed to produce peripheral vasoconstriction and/or increase cardiac output. Aldosterone analogs, such as the mineralocorticoid fludrocortisone (FLORINEF^{®}), have been used in some patients to expand intravascular volume; however the efficacy of these drugs is often unpredictable, and such treatment carries the risk of volume overload, as well as possible development of hypokalemia or hypomagnesemia. Midodrine (PROAMITINE^{®}), a vasoconstrictor, may be used in patients with severe orthostatic unresponsive to other drugs, but this also has undesirable side effects, papillary dilation, piloerection, parasthesias, pruritus, and supine hypertension and is capable of inducing hypertensive crisis.

In addition to the above, orthostatic hypotension can also be classified as "central" type or "peripheral" type. Central type orthostatic hypotension is so classified as arising from, or being directly related to, the central nervous system, particularly the sympathetic nerves. Peripheral type orthostatic hypotension broadly includes non-central types, such as non-neurogenic and iatrogenic orthostatic hypotension, as well as neurogenic orthostatic hypotension related to the peripheral nervous system. As previously noted, it is known that droxidopa is converted to norepinephrine in the body. It is also known that norepinephrine is a catecholamine useful as a hypertensor.

Given the unpredictability and unfavorable side effects associated with the typical treatments for orthostatic hypotension, there remains a need in the art for compositions useful for treating orthostatic hypotension. Furthermore, there remains a need for compositions useful for treating orthostatic hypotension generally, without regard to type. The present invention provides such useful compositions.

### SUMMARY OF THE INVENTION

The present invention provides droxidopa in combination with one or more further pharmaceutically active compounds. The combinations of the invention are particularly useful in the treatment of orthostatic hypotension.

In certain embodiments, the invention provides droxidopa in combination with one or more additional compounds selected from the group consisting of catechol-O-methyltransferase inhibiting compounds, Cholinesterase inhibiting compounds, monoamine oxidase inhibiting compounds, and combinations thereof. In a specific embodiment, the combination comprises droxidopa and one or more catechol-O-methyltransferase inhibiting compounds. A particular embodiment comprises droxidopa in and entacapone or tolcapone.

In another embodiment, the invention comprises droxidopa in combination with one or more Cholinesterase inhibiting compounds. Particularly preferred is droxidopa in combination with pyridostigmine. In still another embodiment, the invention comprises droxidopa in combination one or more monoamine oxidase inhibiting compounds. A specific embodiment comprises droxidopa in combination with nialamide.

In another aspect, the invention provides methods for increasing the half-life of droxidopa in a mammal. Specifically, the method comprises administering the droxidopa to the mammal in combination with one or more additional compounds selected from the group consisting of catechol-O-methyltransferase inhibiting compounds, cholinesterase inhibiting compounds, monoamine oxidase inhibiting compounds, and combinations thereof. In particular embodiments, the half-life of the droxidopa is increased by a specific amount, such as at least 20% or at least 50%. Moreover, the method of the invention can be carried out in a variety of manners, such as by administering the droxidopa and the one or more additional compounds simultaneously or sequentially.

In yet another aspect, the invention provides methods for increasing the volume of distribution of droxidopa in a mammal. In one embodiment, the method comprises administering the droxidopa to the mammal in combination with one or more additional compounds selected from the group consisting of catechol-O-methyltransferase inhibiting compounds, cholinesterase inhibiting compounds, monoamine oxidase inhibiting compounds, and combinations thereof. In particular embodiments, the volume of distribution of the droxidopa is increased by a specific amount, such as at least 20% or at least 50%. Moreover, the method of the invention can be carried out in a variety of manners, such as by administering the droxidopa and the one or more additional compounds simultaneously or sequentially.

According to still another aspect, the invention provides methods of treating certain conditions responsive to droxidopa in combination with one or more further pharmaceutically active compounds. In certain embodiments, the condition treated according to the method is orthostatic hypotension. In one specific embodiment, the invention provides a method of treating orthostatic hypotension comprising administering to a subject in need of treatment for orthostatic hypotension a combination of droxidopa and one or more additional compounds selected from the group consisting of catechol-O-methyltransferase inhibiting compounds, cholinesterase inhibiting compounds, monoamine oxidase inhibiting compounds, and combinations thereof.

### BRIEF DESCRIPTION OF THE DRAWINGS

Having thus described the invention in general terms, reference will now be made to the accompanying drawing, which is not necessarily drawn to scale, and wherein:
FIG. 1 is a graphical representation of the half-life of droxidopa in a mammal when administered alone or in combination according to various embodiments of the invention.

### DETAILED DESCRIPTION

The invention now will be described more fully hereinafter through reference to various embodiments. These embodiments are provided so that this disclosure will be thorough and complete, and will fully convey the scope of the invention to those skilled in the art. Indeed, the invention may be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural referents unless the context clearly dictates otherwise.

The present invention provides combinations of compounds having pharmaceutical activity and being useful in the treatment of specific conditions. In particular, the combinations are useful in the treatment of orthostatic hypotension.

The combinations of the invention generally comprise threo-3-(3,4-dihydroxyphenyl) serine, which is commonly known as droxidopa and has the structure provided below. 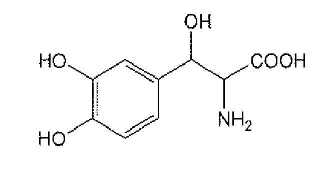 Droxidopa is also known as threo-ß,3-dihydroxy-L-tyrosine, (-)-(2S,3R)-2-amino-3-hydroxy-3-(3,4-dihydroxyphenyl)propionic acid, and threo-dopaserine, as well as the common terms DOPS, threo-DOPS, and L-DOPS. The compound is optically active having D- and L- forms, as well as a racemic DL- form. The L- isomer is generally preferred according to the present invention; however, the invention also encompasses combinations and methods of use incorporating the racemic DL- form of droxidopa. Accordingly, as used throughout the present disclosure, the term "droxidopa" is intended to encompass the L- isomer, as well as the racemic DL- form of droxidopa.

Droxidopa useful according to the invention can be prepared by conventional methods, including methods particularly useful for isolating the L- isomer of droxidopa. See, for example, U.S. Patent No. 3,920,728; U.S. Patent No. 4,319,040; U.S. Patent No. 4,480,109; U.S. Patent No. 4,562,263; U.S. Patent No. 4,699,879; U.S. Patent No. 5,739,387; and U.S. Patent No. 5,864,041, which are incorporated herein by reference.

The present invention also encompasses combinations comprising one or more pharmaceutically acceptable esters, amides, salts, or solvates of droxidopa. Such further compounds can be prepared according to methods that would generally be recognized as useful by the skilled artisan. Non-limiting examples of such compounds would include droxidopa esters that allow for slowed or delayed decarboxylation of droxidopa resulting from hydrolytic or enzymatic degradation of the ester linkage. Moreover, any droxidopa esters, amides, salts, or solvates capable of systematic conversion to norepinephrine would be useful according to the invention.

The combinations of the invention comprise droxidopa and one or more additional compounds. In one particular embodiment, the inventive combination comprises droxidopa and one or more compounds that at least partially inhibit the function of catechol-O-methyltransferase (such compounds being generally referred to as "COMT inhibitors").

Catechol-O-methyltransferase catalyzes the transfer of the methyl group from S-adenosyl-L-methionine to various catechol compounds (*e.g*., catecholamines), including dopamine, epinephrine, norepinephrine, and droxidopa. The COMT enzyme is important in the extraneuronal inactivation of catecholamines and drugs with catechol structures, and is generally one of the most important enzymes involved in the metabolism of catecholamines and their metabolites. It is present in most tissues, including the peripheral and the central nervous system.

Inhibitors of COMT slow metabolism and elimination of catechol compounds. Accordingly, COMT inhibitors can function to increase levels of naturally occurring catechol compounds, as well as alter the pharmacokinetics of administered catechol compounds (such as L-ß-3,4-dihydroxyphenytalanine (L-DOPA), an immediate precursor of dopamine, generally used for symptomatic treatment of Parkinson's disease). Inhibitors of COMT can act peripherally (such as the compound entacapone), while others (such as tolcapone) are capable of crossing the blood-brain barrier and thus acting centrally and peripherally.

While not wishing to be bound by theory, it is believed that peripheral hypotension and central hypotension each can be more effectively treated using COMT inhibitors having type-specific activity. For example, peripheral hypotension may be more effectively treated using COMT inhibitors having only a peripheral activity while central hypotension may be more effectively treated using COMT inhibitors having a central activity.

Any compound generally recognized as being a COMT inhibitor can be used according to the invention. Non-limiting examples of COMT inhibitors useful in combination with droxidopa according to the invention include the following: [(E)-2-cyano-N,N-diethyl-3-(3,4-dihydroxy-5-nitrophenyl)propenamide], also called entacapone (COMTAN^{®}); 4-dihydroxy-4'-methyl-5-nitrobenzophenone, also called tolcapone (TASMAR^{®}); and 3-(3,4-dihydroxy-5-nitrophenyl)methylene-2,4-pentanedione, also called nitecapone. In addition to the above examples, U.S. Patent No. 6,512,136 (the disclosure of which is incorporated herein by reference) describes various substituted 2-phenyl-1-(3,4-dihydroxy-5-nitrophenyl)-1-ethanone compounds that may also be useful as COMT inhibitors according to the present invention. Likewise, U.S. Patent No. 4,963,590; GB 2 200 109; U.S. Patent No. 6,150,412; and EP 237 929, each describes groups of COMT inhibiting compounds that could be useful according to the present invention, and the disclosure of each of the above-noted documents is incorporated herein by reference.

According to another embodiment of the invention, the combination comprises droxidopa and one or more compounds that at least partially inhibit the function of cholinesterase. Such cholinesterase inhibiting compounds may also be referred to as anticholinesterase compounds. Cholinesterase inhibiting compounds can be reversible or non-reversible. The present invention preferably encompasses any compounds that may be considered reversible cholinesterase inhibitors (either competitive or non-competitive inhibitors). Non-reversible cholinesterase inhibitors generally find use as pesticides (such as diazinon and Sevin) and chemical weapons (such as tabin and sarin) and are not preferred according to the present invention.

Cholinesterase inhibitors are understood to include compounds that increase levels of acetylcholine, generally by reducing or preventing the activity of chemicals involved in the breakdown of acetylcholine, such as acetylcholinesterase. Cholinesterase inhibitors may also include compounds having other mechanisms of action, such as stimulating release of acetylcholine and enhancing response of acetylcholine receptors. Moreover, cholinesterase inhibitors may act by enhancing ganglionic transmission. For example pyridostigmine bromide has been found to enhance ganglionic transmission and thereby potentially ameliorate orthostatic hypotension without worsening supine hypertension. See Singer W., Sandroni P., Opfer-Gehrking T.L., Suarez G.A., Klein C.M., Hines S., O'Brien P.C., Slezak J., and Low P.A., (2006) Arch. Neurol. 63(4): 513-518, which is incorporated herein by reference.

Any compound generally recognized as being a cholinesterase inhibitor (or an anticholinesterase compound) may be useful according to the present invention. Non-limiting examples of cholinesterase inhibitors useful in combination with droxidopa according to the invention include the following: 3-dimethylcarbamoyloxy-1-methylpyridinium, also called pyridostigmine (MESTINON^{®} or Regonol); (±)-2,3-dihydro-5,6-dimethoxy-2-[[1-(phenylmethyl)-4-piperidinyl]methyl]-1*H*-inden-1-one, also called donepezil (ARICEPT^{®}); (S)-N-ethyl-3-((1-dimethyl-amino)ethyl)-N-methylphenyl-carbamate, also called rivastigmine (Exelon); (4aS,6R,8aS)-4a,5,9,10,11,12-hexahydro-3-methoxy-11-methyl-6H-benzofuro[3a, 3, 2ef ][2]benzazepin-6-ol, also called galantamine (REMINYL^{®} or RAZADYNE^{®}); 9-amino-1,2,3,4-tetrahydroacridine, also called tacrine (COGNEX^{®}); (*m-*hydroxyphenyl) trimethylammonium methylsulfate dimethylcarbamate, also called neostigmine; 1-hydroxy-2,2,2-trichloroethylphosphonic acid dimethyl ester, also called metrifonate or trichlorofon; 1,2,3,3A,8,8A-hexahydro-1,3a,8-trimethylpyrrolo-[2,3-b]-indole-5-ol methylcarbamate ester, also called physostigmine; [Oxalylbis(iminoethylene)]-bis-[(o-chlorobenzyl) diethylammonium] dichloride, also called ambenonium (MYTELASE^{®} or MESTINON^{®}); ethyl (*m-*hydroxyphenyl) dimethylammonium, also called edrophonium (ENLON^{®}); demarcarium; thiaphysovenine; phenserine; and cymserine.

More generally, compounds useful as cholinesterase inhibitors according to the invention can comprise carbamate compounds, particularly phenylcarbamates, oganophosphate compounds, piperidines, and phenanthrine derivatives. The invention further comprises cholinesterase inhibitors that are carbamoyl esters, as disclosed in U.S. Published Patent Application No. 2005/0096387, which is incorporated herein by reference.

The above groups of compounds, and specific compounds, are provided to exemplify the types of cholinesterase inhibitors that are useful according to the invention and should not be viewed as limiting the scope of the invention. In fact, the invention can incorporate various further cholinesterase inhibitors, including compounds described in the following documents, the disclosures of which are incorporated herein by reference: Brzostowska, Malgorzata, et al. "Phenylcarbamates of (-)-Eseroiine, (-)-N1-Noreseroline and (-)-Physovenol: Selective Inhibitors of Acetyl and, or Butyrylcholinesterase." Medical Chemistry Research. (1992) Vol. 2, 238-246; Flippen-Anderson, Judith L., et al. "Thiaphysovenol Phenylcarbamates: X-ray Structures of Biologically Active and Inactive Anticholinesterase Agents." Heterocycles. (1993) Vol. 36, No. 1; Greig, Nigel H., et al. "Phenserine and Ring C Hetero-Analogues: Drug Candidates for the Treatment of Alzheimer's Disease." Medicinal Research Reviews. (1995) Vol. 15, No. 1, 3-31; He, Xiao-shu, et al. "Thiaphysovenine and Carbamate Analogues: A New Class of Potent Inhibitors of Cholinesterases." Medical Chemistry Research. (1992) Vol. 2, 229-237; Lahiri, D.K., et al. "Cholinesterase Inhibitors, ß-Amyloid Precursor Protein and Amyloid ß-Peptides in Alzheimer's Disease." Acta Neurologica Scandinavia. (December 2000) Vol. 102 (s176), 60-67; Pei, Xue-Feng, et al. "Total Synthesis of Racemic and Optically Active Compounds Related to Physostigimine and Ring-C Heteroanalogues from 3[-2'-(Dimethylamino0ethyl]-2,3-dihydro-5-methoxy-1, 3-dimentyl-1H-andal-2-ol." Helvetica Chimica ACTA. (1994) Vol.77; Yu, Qian-sheng, et al. "Total Syntheses and Anticholinesterase Activities of (3aS)-N (8)-Norphysostigmine, (3aS)-N (8)-Norphenserine, Their Antipodal Isomers, and Other N (8)-Substituted Analogues." J. Med. Chem. (1997) Vol. 40, 2895-2901; and Yu, Q.S., et al. "Novel Phenserine-Based-Selective Inhibitors of Butyrylcholinesterase for Alzheimer's Disease." Reprinted with permission from J. Med. Chem., May 20, 1999,42, 1855-1861.

According to yet another embodiment of the invention, the combination comprises droxidopa and one or more compounds that at least partially inhibit the function of monoamine oxidase. Monoamine oxidase inhibitors (MAOIs) comprise a class of compounds understood to act by inhibiting the activity of monoamine oxidase, an enzyme generally found in many parts of the body, including neuronal tissue, the liver, and other parts of the gut. Monoamine oxidase function to break down monoamine compounds, typically through deamination.

There are two isoforms of monoamine oxidase inhibitors, MAO-A and MAO-B. The MAO-A isoform preferentially deaminates monoamines typically occurring as neurotransmitters (*e.g*., serotonin, melatonin, epinephrine, norepinephrine, and dopamine). Thus, MAOIs have been historically prescribed as antidepressants and for treatment of other social disorders, such as agoraphobia and social anxiety. The MAO-B isoform preferentially deaminates phenylethylamine and trace amines. Dopamine is equally deaminated by both isoforms. MAOIs may by reversible or non-reversible and may be selective for a specific isoform. For example, the MAOI moclobemide (also known as Manerix or Aurorix) is known to be approximately three times more selective for MAO-A than MAO-B.

When taken orally, MAOIs are known to inhibit catabolism of dietary amines, including tyramine. Accordingly, combining MAOIs with sufficient tyramine intake can cause hypertension. As tyramine is a component in the formation of norepinephrine, bodily tyramine concentrations can directly affect the amount of norepinephrine present in the body. As previously noted, MAOIs inhibit monoamine oxidase activity, thereby causing an increase in bodily norepinephrine levels. Excessive amounts of norepinephrine can cause increases in blood pressure, sometimes to dangerously high levels. However, in some circumstances, such as treatment of orthostatic hypotension, increasing norepinephrine levels can be desirable.

Any compound generally recognized as being an MAOI may be useful according to the present invention. Non-limiting examples of MAOIs useful in combination with droxidopa according to the invention include the following: isocarboxazid (MARPLAN^{®}); moclobemide (Aurorix, Manerix, or Moclodura); phenelzine (NARDIL^{®}); tranylcypromine (PARNATE^{®}); selegiline (ELDEPRYL^{®}, EMSAM^{®}, or 1-deprenyl); lazabemide; nialamide; iproniazid (marsilid, iprozid, ipronid, rivivol, or propilniazida); iproclozide; toloxatone; harmala; brofaromine (Consonar); bentnoxin (Neuralex); and certain tryptamines, such as 5-MeO-DMT (5-Methoxy-N,N-dimethyltryptamine) or 5-MeO-AMT (5-methoxy-α-methyltryptamine).

The COMT inhibiting compounds, cholinesterase inhibiting compounds, and MAOI compounds provided above are not intended to limit the scope of compounds provided according to the invention for use in combination with droxidopa. On the contrary, the invention encompasses multiple variants of the above compounds. In particular, the invention also encompasses pharmaceutically acceptable esters, amides, salts, or solvates of the various compounds described herein.

Biologically active variants of COMT inhibitors, cholinesterase inhibitors, and MAOIs set forth above are particularly also encompassed by the invention. Such variants should retain the biological activity of the original compounds (*i.e*., the ability to inhibit COMT activity, Cholinesterase activity, or monoamine oxidase activity). Such activity may be evaluated using standard testing methods and bioassays recognizable by the skilled artisan in the field as generally being useful for identifying such activity.

According to one embodiment of the invention, suitable biologically active variants comprise analogues and derivatives of the COMT inhibitors, cholinesterase inhibitors, and MAOIs described above. As used herein, an "analogue" refers to a compound in which one or more individual atoms or functional groups have been replaced, either with a different atom or a different functional, generally giving rise to a compound with similar properties. Indeed, a single compound, such as those described above, may give rise to an entire family of analogues having similar activity and, therefore, usefulness according to the present invention. Likewise, a single compound, such as those described above, may represent a single family member of a greater class of compounds useful according to the present invention. Accordingly, the present invention fully encompasses not only the compounds described above, but analogues of such compounds, particularly those identifiable by methods commonly known in the art and recognizable to the skilled artisan.

A "derivative", as used herein, comprises a compound that is formed from a similar, beginning compound by attaching another molecule or atom to the beginning compound. Further, derivatives, according to the invention, encompass one or more compounds formed from a precursor compound through addition of one or more atoms or molecules or through combining two or more precursor compounds.

The present invention also includes stereoisomers of the COMT inhibitors, cholinesterase inhibitors, and MAOIs described herein, where applicable, either individually or admixed in any proportions. Stereoisomers may include, but are not limited to, enantiomers, diastereomers, racemic mixtures and combinations thereof. Such stereoisomers can be prepared and separated using conventional techniques, either by reacting enantiomeric starting materials, or by separating isomers of compounds of the present invention. Isomers may include geometric isomers. Examples of geometric isomers include, but are not limited to, cis isomers or trans isomers across a double bond. Other isomers are contemplated among the compounds of the present invention. The isomers may be used either in pure form or in admixture with other isomers of the compounds described herein.

The present invention further includes prodrugs and active metabolites of the COMT inhibitors, cholinesterase inhibitors, and MAOIs of the invention. A prodrug includes any compound which, when administered to a mammal, is converted in whole or in part to a compound of the invention. An active metabolite is a physiologically active compound which results from the metabolism of a compound of the invention, or a prodrug thereof, when such compound or prodrug is administered to a mammal.

According to another aspect, the invention is directed to methods of treating certain conditions. For example, one embodiment of the invention is directed to methods of treating orthostatic hypotension. Preferably, the method comprises administering to a subject in need of treatment for orthostatic hypotension a combination of droxidopa and one or more additional compounds selected from the group consisting of COMT inhibiting compounds, cholinesterase inhibiting compounds, MAO inhibiting compounds, and combinations thereof.

In one specific embodiment, the invention provides a method of treating orthostatic hypotension comprising administering to a subject in need of treatment for orthostatic hypotension a combination of droxidopa and one or more COMT inhibiting compounds. The combination can comprise droxidopa and any of the COMT inhibitors described herein. In a particularly preferred embodiment, the combination comprises droxidopa and entacapone. In another preferred embodiment, the combination used in the method comprises droxidopa and tolcapone. In yet another preferred embodiment of the invention, the combination used in the method comprises droxidopa and nitecapone.

According to another embodiment, the invention provides a method of treating orthostatic hypotension comprising administering to a subject in need of treatment for orthostatic hypotension a combination comprising droxidopa and one or more cholinesterase inhibiting compounds. The combination can comprise droxidopa and any of the cholinesterase inhibitors described herein. In a particularly preferred embodiment, the combination used in the method of the invention comprises droxidopa and pyridostigmine.

According to still another embodiment, the invention provides a method of treating orthostatic hypotension comprising administering to a subject in need of treatment for orthostatic hypotension a combination comprising droxidopa and one or more monoamine oxidase inhibiting compounds. The combination can comprise droxidopa and any of the MAOIs described herein. In a particular embodiment, the combination used in the method of the invention comprises droxidopa and selegiline. In another embodiment of the invention, the combination used in the method of the invention comprises droxidopa and moclobemide. In still another embodiment of the invention, the combination used in the method of the invention comprises droxidopa and nialamide.

The methods of the invention are particularly useful in light of the benefits provided by the inventive combinations described herein. As previously noted, droxidopa is converted to norepinephrine by the action of the aromatic L-amino acid decarboxylase. Droxidopa is particularly useful for treating orthostatic hypotension because of its ability to increase norepinephrine levels via the noted conversion process, thereby increasing blood pressure. The various combinations of the invention, in certain embodiments, are particularly beneficial for treating orthostatic hypotension because the combination of the additional component with droxidopa has a conserving effect on the droxidopa, as well as a potential effect on redistribution within pharmacokinetic compartments.

As previously noted, catechol-O-methyltransferase is directly involved in the metabolism of catecholamines, including dopamine, epinephrine, norepinephrine, and droxidopa. Accordingly, by providing droxidopa in combination with a COMT inhibitor, the effect of the droxidopa to affect blood pressure is conserved. Specifically, by inhibiting the action of COMT, the COMT inhibiting compound slows or delays the metabolism of droxidopa (as well as norepinephrine itself) and particularly increases the half-life (T_{1/2}) of the administered droxidopa. This is particularly beneficial in that it allows for reduced dosages of droxidopa without limiting effective treatment of orthostatic hypotension. Further, the combination of the COMT inhibitor with droxidopa may be effective for increasing the duration of the droxidopa activity (*i.e.,* increasing the duration of norepinephrine activity), which may allow for a reduction in dosing frequency of the droxidopa. Still further, the combination of a peripherally-acting COMT inhibitor with droxidopa may be effective for facilitating the redistribution of droxidopa from a peripheral compartment to the central compartment, which could also lead to an increase in droxidopa half-life.

The combination of droxidopa with an MAOI has a similar effect of conserving bodily norepinephrine levels. In particular embodiments, the MAOI inhibits the action of monoamine oxidase in breaking down norepinephrine, including that formed from the conversion of droxidopa. Co-administration of a MAOI compound with droxidopa thus increases droxidopa half-life. This is again particularly beneficial in allowing for a reduction in dosing frequency of the droxidopa.

In certain embodiments, the combination of droxidopa with cholinesterase inhibitors is particularly effective arising from synergistic properties. As previously noted, certain cholinesterase inhibitors (such as pyridostigmine) have been found to enhance ganglionic transmission, thereby directly affecting blood pressure and providing some degree of treatment for orthostatic hypotension. The synergistic effect of the cholinesterase inhibitor with droxidopa can therefore be envisioned. For example, in a specific embodiment, pyridostigmine could be combined with droxidopa, the pyridostigmine enhancing ganglionic neurotransmission while the droxidopa acts to load the postganglionic neuron with norepinephrine.

As pointed out above, the present invention is particularly useful for increasing the half-life of droxidopa when administered to a subject, particularly a mammal. Generally, such increased half-life is provided by administering the droxidopa in combination with one or more additional compounds as described herein. Moreover, as described below, such combined administration can be via a variety of methods (*e.g*., simultaneous administration or sequential administration).

The compounds for combination with droxidopa comprise inhibitors of a number of enzymes that are involved in the metabolism of sympathetic neurotransmitters (such as COMT and MAO) and parasympathetic neurotransmitters (such as cholinesterase). Thus, the invention is useful for increasing droxidopa half-life via a variety of pathways. For example, COMT inhibitors, such as entacapone, work peripherally to block the metabolism of droxidopa to 3-OM-droxidopa (the major metabolite of droxidopa), thus increasing residence of droxidopa in the body. According to another mode of action, MAO catalyzes the major inactivation pathway of the catecholamine neurotransmitters (*e.g*., epinephrine, norepinephrine, and dopamine) and is not typically considered to be a major metabolic pathway for droxidopa. Thus, it is particularly surprising according to the present invention that co-administration of droxidopa with a MAOI, such as nialamide, was effective for increasing the half-life of droxidopa after administration to a mammalian subject. Similarly, cholinesterase compounds would generally not be expected to affect droxidopa metabolism. Nevertheless, as surprisingly illustrated in the Example, combining droxidopa with the cholinesterase inhibitor pyridostigmine increased droxidopa half-life by greater than 30%. This increase may be a result of an increase in the volume of distribution of droxidopa, which is also a surprising effect arising from the present invention. The effect of increasing droxidopa half-life post administration is further illustrated in the Example and is illustrated in FIG. 1. The data for use in preparing FIG. 1 was obtained in the evaluation described in the Example.

In certain embodiments, the combinations of the invention are effective for increasing droxidopa half-life by a specific percent in relation to the half-life of droxidopa when administered under the same conditions but without the additional compounds. In preferred embodiments, combining the one or more further compounds with the droxidopa according to the invention increases the half-life of the droxidopa by at least 10%, at least 20%, at least 30%, at least 40%, or at least 50%. In a particularly preferred embodiment, combining the one or more further compounds with the droxidopa at least doubles the half-life of the droxidopa (*i.e*., increases the half-life by two-fold).

The drug combinations of the invention also affect the apparent volume of distribution of droxidopa in comparison to administration of droxidopa alone. Apparent volume of distribution is used to describe how well a drug is distributed to the tissues. A large volume of distribution implies wide distribution, or extensive tissue binding, or both. For drugs that are highly tissue-bound, comparatively little of a dose remains in the circulation to be measured, and thus plasma concentration is low and volume of distribution is high. Drugs that remain in the circulation (such as ionized drugs) tend to have a low volume of distribution. The value is particularly important for determining the drug fraction available to the organs of elimination.

The effect of the inventive combinations on apparent volume of distribution is illustrated in the Examples below, particularly in Table 5. Such an increased apparent volume of distribution potentially arises from a redistribution af droxidopa (and thus norepinephrine) from the peripheral compartment to the central compartment (*i.e*., the central nervous system). Such an effect may particularly arise from the combination with COMT inhibitors, which are known to work peripherally in the body. Thus, the combinations of the invention may be particularly useful at increasing the central effect of droxidopa and norepinephrine in mammals. An increase in the volume of distribution can result in an increase in elimination half-life, since less of the drug may be available to organs of elimination.

In light of the above, the present invention, in specific embodiments, further provides a method for increasing the volume of distribution of droxidopa in a mammal. Preferably, the method comprises administering the droxidopa to the mammal in combination with one or more additional compounds selected from the group consisting of catechol-O-methyltransferase inhibiting compounds, cholinesterase inhibiting compounds, monoamine oxidase inhibiting compounds, and combinations thereof. Further, combining the one or more further compounds with the droxidopa according to the invention preferentially increases the apparent volume of distribution of the droxidopa by at least 10%, at least 20%, at least 30%, at least 40%, or at least 50%.

The combinations of the invention are further beneficial in that it is possible to prepare combinations particularly designed to affect plasma norepinephrine concentration. As illustrated in the Example below, administration of droxidopa alone is effective for increasing plasma norepinephrine concentration in mammals. The effect of droxidopa on plasma norepinephrine concentration is either increased or reduced depending upon the compound with which the droxidopa is combined. For example co-administration of droxidopa with compounds having peripheral activity to block the conversion of droxidopa to norepinephrine can function to lessen the increased plasma norepinephrine concentration arising from the droxidopa. This peripheral effect can also function, however, to redistribute droxidopa to the central nervous system, resulting in enhanced central norepinephrine levels.

Entacapone is an example of a COMT inhibitor that works peripherally to block the metabolism of both droxidopa and norepinephrine. As seen in Table 5, administration of droxidopa combined with entacapone increased plasma norepinephrine concentration beyond the effect of droxidopa alone. Such an effect can be useful for increasing peripheral norepinephrine levels for specific indications. This could likewise increase norepinephrine levels in the central nervous system.

The compounds described herein for use in the combinations of the invention can be administered in the form of an ester, amide, salt, solvate, prodrug, metabolite, derivative, or the like, provided it maintains pharmacological activity according to the present invention. Esters, amides, salts, solvates, prodrugs, and other derivatives of the compounds of the present invention may be prepared according to methods generally known in the art, such as, for example, those methods described by J. March, Advanced Organic Chemistry: Reactions, Mechanisms and Structure, 4th Ed. (New York: Wiley-Interscience, 1992).

Examples of pharmaceutically acceptable salts of the compounds useful according to the invention include acid addition salts. Salts of non-pharmaceutically acceptable acids, however, may be useful, for example, in the preparation and purification of the compounds. Suitable acid addition salts according to the present invention include organic and inorganic acids. Preferred salts include those formed from hydrochloric, hydrobromic, sulfuric, phosphoric, citric, tartaric, lactic, pyruvic, acetic, succinic, fumaric, maleic, oxaloacetic, methanesulfonic, ethanesulfonic, p-toluenesulfonic, benzesulfonic, and isethionic acids. Other useful acid addition salts include propionic acid, glycolic acid, oxalic acid, malic acid, malonic acid, benzoic acid, cinnamic acid, mandelic acid, salicylic acid, and the like. Particular example of pharmaceutically acceptable salts include, but are not limited to, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxyenzoates, phthalates, sulfonates, xylenesulfonates, phenylacetates, phenylpropionates, phenylbutyrates, citrates, lactates, γ-hydroxybutyrates, glycolates, tartrates, methanesulfonates, propanesulfonates, naphthalene-1-sulfonates, naphthalene-2-sulforiates, and mandelates.

An acid addition salt may be reconverted to the free base by treatment with a suitable base. Preparation of basic salts of acid moieties which may be present on a compound useful according to the present invention may be prepared in a similar manner using a pharmaceutically acceptable base, such as sodium hydroxide, potassium hydroxide, ammonium hydroxide, calcium hydroxide, triethylamine, or the like.

Esters of the compounds useful in combinations according to the present invention may be prepared through functionalization of hydroxyl and/or carboxyl groups that may be present within the molecular structure of the compound. Amides and prodrugs may also be prepared using techniques known to those skilled in the art. For example, amides may be prepared from esters, using suitable amine reactants, or they may be prepared from anhydride or an acid chloride by reaction with ammonia or a lower alkyl amine. Moreover, esters and amides of compounds of the invention can be made by reaction with a carbonylating agent (*e.g*., ethyl formate, acetic anhydride, methoxyacetyl chloride, benzoyl chloride, methyl isocyanate, ethyl chloroformate, methanesulfonyl chloride) and a suitable base (*e.g*., 4-dimethylaminopyridine, pyridine, triethylamine, potassium carbonate) in a suitable organic solvent (*e.g*., tetrahydrofuran, acetone, methanol, pyridine, N,N-dimethylformamide) at a temperature of 0 °C to 60 °C. Prodrugs are typically prepared by covalent attachment of a moiety, which results in a compound that is therapeutically inactive until modified by an individual's metabolic system. Examples of pharmaceutically acceptable solvates include, but are not limited to, compounds according to the invention in combination with water, isopropanol, ethanol, methanol, DMSO, ethyl acetate, acetic acid, or ethanolamine.

In the case of solid formulations, it is understood that the compounds used in the combinations of the invention may exist in different forms. For example, the compounds may exist in stable and metastable crystalline forms and isotropic and amorphous forms, all of which are intended to be within the scope of the present invention.

If a compound useful according to the invention is a base, the desired salt may be prepared by any suitable method known to the art, including treatment of the free base with an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, pyranosidyl acids such as glucuronic acid and galacturonic acid, alpha-hydroxy acids such as citric acid and tartaric acid, amino acids such as aspartic acid and glutamic acid, aromatic acids such as benzoic acid and cinnamic acid, sulfonic acids such a p-toluellesulfonic acid or ethanesulfonic acid, or the like.

If a compound used in the inventive combinations is an acid, the desired salt may be prepared by any suitable method known to the art, including treatment of the free acid with an inorganic or organic base, such as an amine (primary, secondary or tertiary), an alkali metal or alkaline earth metal hydroxide or the like. Illustrative examples of suitable salts include organic salts derived from amino acids such as glycine and arginine, ammonia, primary, secondary and tertiary amines, and cyclic amines such as piperidine, morpholine and piperazine, and inorganic salts derived from sodium, calcium, potassium, magnesium, manganese, iron, copper, zinc, aluminum and lithium.

Delivery of a therapeutically effective amount of a combination according to the invention may be obtained via administration of a therapeutically effective dose of the combination. By "therapeutically effective amount" or "dose" is meant a concentration of droxidopa and one or more of a COMT inhibiting compound, a cholinesterase inhibiting compound, and a monoamine oxidase inhibiting compound, that is sufficient to elicit the desired therapeutic effect according to the methods of treatment described herein. Accordingly, in one embodiment, a therapeutically effective amount is an amount effective to treat orthostatic hypotension.

It is contemplated that the combinations of the invention comprising droxidopa and a COMT inhibitor, a cholinesterase inhibitor, or an MAOI, will be administered to a subject (*i.e*., a mammal, preferably a human) in therapeutically effective amounts. That is, the combinations will be administered in an amount sufficient to effect treatment according to the various therapeutic methods of the invention. The effective amount of the combinations would be expected to vary according to the weight, sex, age, and medical history of the subject. Of course, other factors could also influence the effective amount of the composition to be delivered. Methods to determine efficacy and dosage are known to those skilled in the art. See, for example, Isselbacher et al. (1996) Harrison's Principles of Internal Medicine 13 ed., 1814-1882, herein incorporated by reference.

In certain embodiments, a therapeutically effective amount of droxidopa comprises about 10 mg to about 3 g. Such therapeutically effective amount represents an amount of droxidopa that would be provided in a single dose when used as part of a combination according to the invention. It is understood that when the droxidopa is provided as a salt, ester, amide, or other pharmaceutically acceptable form, the amount of the pharmaceutical form of droxidopa can vary to the extent necessary to deliver a therapeutically effective amount of droxidopa. Further, as the therapeutically effective amount of droxidopa is provided as an amount for a single dose, the dosage amounts indicated herein do not necessarily represent the maximum amount of droxidopa that may be administered over the course of a 24 hour period since it is possible that multiple doses of the combination may be indicated for treatment of various conditions.

In further embodiments, the therapeutically effective amount of droxidopa can encompass varying ranges, and the appropriate range could be determined based upon the condition being treated and the one or more additional compounds with which the droxidopa is combined. In specific embodiments, a therapeutically effective amount of droxidopa comprises about 10 mg to about 2 g, about 10 mg to about 1 g, about 20 mg to about 900 mg, about 30 mg to about 850 mg, about 40 mg to about 800 mg, about 50 mg to about 750 mg, about 60 mg to about 700 mg, about 70 mg to about 650 mg, about 80 mg to about 600 mg, about 90 mg to about 550 mg, about 100 mg to about 500 mg, about 100 mg to about 400 mg, or about 100 mg to about 300 mg.

In yet other embodiments, a therapeutically effective amount of droxidopa can be even greater, such as when provided as a sustained-, extended-, or continuous-release formulation. As understood in the art, such formulations provide an increased drug amount in a single dosage form that slowly releases the drug over time. A therapeutically effective amount of droxidopa for use in such a formulation can be calculated in light of the effective amounts described above and the determined frequency of dosing that would otherwise be necessary to treat a given condition.

A therapeutically effective amount of the one or more additional compounds that are combined with droxidopa according to the invention can be determined in relation to the amount of droxidopa included in the dosage form and the desired ratio of droxidopa to the additional compound(s). Advantageously, the present invention allows for great flexibility in formulating combinations. For example, the conserving effects provided by the one or more additional compounds can allow for using droxidopa in a lesser amount and still achieve the same, or better, therapeutic effects achieved using droxidopa alone. Likewise, it is possible to increase the therapeutic effects of droxidopa by using an amount of the one or more additional compounds that is less than the typically recommended dosage for the one or more additional compounds.

In one embodiments, the ratio of droxidopa to the one or more additional compounds is in the range of about 500: 1 to about 1:10. In further embodiments, the droxidopa:additional compound(s) ratio is in the range of about 250:1 to about 1:5, about 100:1 to about 1:2, about 80:1 to about 1:1, about 50:1 to about 2:1, or about 20:1 to about 3:1.

The one or more additional compounds combined with droxidopa according to the invention can be included in amount typically recommended for use of the compounds alone for other indications. However, as noted above, it is possible according to the invention to use the additional compound(s) in amounts that are less than typically recommended. In certain embodiments, a therapeutically effective amount of the one or more additional compounds to be combined with droxidopa is in the range of about 1 mg to about 200 mg. Of course, this range is exemplary and could vary depending upon the amount of droxidopa included in the combination and the desired ratio of the compounds in the combination, as described above.

In another embodiment of the invention, the combination of the invention is administered intermittently. By "intermittent administration" is intended administration of a therapeutically effective dose of a combination according to the invention, followed by a time period of discontinuance, which is then followed by another administration of a therapeutically effective dose, and so forth. Administration of the therapeutically effective dose may be achieved in a continuous manner, as for example with a sustained-release formulation, or it may be achieved according to a desired daily dosage regimen, as for example with one, two, three, or more administrations per day. By "time period of discontinuance" is intended a discontinuing of the continuous sustained-released or daily administration of the combination. The time period of discontinuance may be longer or shorter than the period of continuous sustained-release or daily administration. During the time period of discontinuance, the level of the individual components of the combination in the relevant tissue is substantially below the maximum level obtained during the treatment. The preferred length of the discontinuance period depends on the concentration of the effective dose and the form of composition used. The discontinuance period can be at least 2 days, at least 4 days or at least 1 week. In other embodiments, the period of discontinuance is at least 1 month, 2 months, 3 months, 4 months or greater. When a sustained-release formulation is used, the discontinuance period must be extended to account for the greater residence time of the combination in the body. Alternatively, the frequency of administration of the effective dose of the sustained-release formulation can be decreased accordingly. An intermittent schedule of administration of a combination of the invention can continue until the desired therapeutic effect, and ultimately treatment of the disease or disorder, is achieved.

As the invention comprises a combination of at least two pharmaceutically active agents (e.g., droxidopa and a COMT inhibitor, cholinesterase inhibitor, or MAOI), it follows that administration of the composition comprises administering droxidopa in combination with one or more further compounds (*i.e*., co-administration). Accordingly, it is recognized that the droxidopa and the additional pharmaceutically active compound can be administered in a fixed combination as a single dosage unit (*i.e.,* a single pharmaceutical formulation that contains both active materials). Alternatively, the droxidopa component may be administered simultaneously with the additional pharmaceutically active compound (*i.e*., separate dosage units administered at the same time). In another embodiment, the droxidopa component and the additional pharmaceutically active compound are administered sequentially (*i.e*., administration of the droxidopa begins shortly after the end of the administration of the additional pharmaceutically active compound or, alternatively, administration of the droxidopa component precedes the administration of the additional pharmaceutically active compound). In such sequential administration, the amount of time between administrations of the multiple components can vary. One of skill in the art will recognize that the most preferred method of administration will allow the desired therapeutic effect, for example, relief from orthostatic hypotension. An example of a single dosage unit according to the invention would be a single pharmaceutical formulation that includes droxidopa and the one or more additional compounds together (*e.g*., such as in a single pill). An example of separate dosage units would be a first pharmaceutical formulation that includes droxidopa and a distinct pharmaceutical formulation that includes the one or more additional compounds (*e.g*., a blister pack with droxidopa provided in a fist pill and the one or more additional compounds provided in a second pill, wherein the second pill could be administered simultaneously or sequentially with the first pill).

In light of the flexibility provided for administering the combinations of the invention, it is understood that the use of the term "pharmaceutical formulation" in the present application is not necessarily intended to limit the scope of the invention to a single dosage unit with all active ingredients provided therein. Rather, the term pharmaceutical formulation can more broadly encompass all of the various administrative routines described herein (*e.g.,* single dosage units and well as separate dosage units for simultaneous or sequential administration).

While it is possible for the individual compound used in the combinations of the present invention to be administered in the raw chemical form, it is preferred for the compounds to be delivered as a pharmaceutical formulation. Accordingly, there are provided by the present invention pharmaceutical compositions comprising droxidopa and at least one additional compound selected from the group consisting of COMT inhibiting compounds, cholinesterase inhibiting compounds, and MAOIs. As such, the formulations of the present invention comprise the pharmaceutically active compounds, as described above, or pharmaceutically acceptable esters, amides, salts, or solvates thereof, together with one or more pharmaceutically acceptable carriers therefore, and optionally, other therapeutic ingredients. Further, the inventive compositions can be prepared and delivered in a variety of forms. For example, the composition can comprise a single formulation containing all of the active ingredients (*e.g*., a single dosage unit of droxidopa and a COMT inhibitor, a cholinesterase inhibitor, an MAOI, or a combination thereof). Alternately, the composition can comprise multiple formulations comprising separate active ingredients but intended to be administered simultaneously, in succession, or in otherwise close proximity of time (*e.g*., separate dosage units of droxidopa and the one or more additional compounds).

By "pharmaceutically acceptable carrier" is intended a carrier that is conventionally used in the art to facilitate the storage, administration, and/or the healing effect of the agent. Carriers should be acceptable in that they are compatible with any other ingredients of the formulation and not harmful to the recipient thereof. A carrier may also reduce any undesirable side effects of the agent. Such carriers are known in the art. See, Wang et al. (1980) J. Parent. Drug Assn. 34(6):452-462, herein incorporated by reference in its entirety.

Formulations of the present invention may include short-term, rapid-onset, rapid-offset, controlled release, sustained release, delayed release, and pulsatile release formulations, providing the formulations achieve administration of a compound as described herein. See Remington's Pharmaceutical Sciences (18th ed.; Mack Publishing Company, Eaton, Pennsylvania, 1990), herein incorporated by reference in its entirety.

Pharmaceutical formulations according to the present invention are suitable for various modes of delivery, including oral, parenteral (including intravenous, intramuscular, subcutaneous, intradermal, and transdermal), topical (including dermal, buccal, and sublingual), and rectal administration. The most useful and/or beneficial mode of administration can vary, especially depending upon the condition of the recipient and the disorder being treated.

The pharmaceutical formulations may be conveniently made available in a unit dosage form, whereby such formulations may be prepared by any of the methods generally known in the pharmaceutical arts. Generally speaking, such methods of preparation comprise combining (by various methods) the active compounds of the invention, such as droxidopa and a COMT inhibitor, cholinesterase inhibitor, or MAOI (or pharmaceutically acceptable esters, amides salts, or solvates thereof) with a suitable carrier or other adjuvant, which may consist of one or more ingredients. The combination of the active ingredients with the one or more adjuvants is then physically treated to present the formulation in a suitable form for delivery (*e.g*., shaping into a tablet or forming an aqueous suspension).

Pharmaceutical formulations according to the present invention suitable as oral dosage may take various forms, such as tablets, capsules (including liquid-filled hard gelatin capsules and softgels), caplets, and wafers (including rapidly dissolving or effervescing), each containing a predetermined amount of the active agent. The formulations may also be in the form of a powder or granules, a solution or suspension in an aqueous or non-aqueous liquid, and as a liquid emulsion (oil-in-water and water-in-oil). The active agents may also be delivered as a powder for reconstitution, bolus, electuary, or paste. It is generally understood that methods of preparations of the above dosage forms are generally known in the art, and any such method would be suitable for the preparation of the respective dosage forms for use in delivery of the compositions according to the present invention.

A tablet containing a combination of the compounds according to the present invention may be manufactured by any standard process readily known to one of skill in the art, such as, for example, by compression or molding, optionally with one or more adjuvant or accessory ingredient. The tablets may optionally be coated or scored and may be formulate so as to provide slow or controlled release of the active agents.

Adjuvants or accessory ingredients for use in the formulations of the present invention can include any pharmaceutical ingredient commonly deemed acceptable in the art, such as binders, fillers, lubricants, disintegrants, diluents, surfactants, stabilizers, preservatives, flavoring and coloring agents, and the like. Binders are generally used to facilitate cohesiveness of the tablet and ensure the tablet remains intact after compression. Suitable binders include, but are not limited to: starch, polysaccharides, gelatin, polyethylene glycol, propylene glycol, waxes, and natural and synthetic gums. Acceptable fillers include silicon dioxide, titanium dioxide, alumina, talc, kaolin, powdered cellulose, and microcrystalline cellulose, as well as soluble materials, such as mannitol, urea, sucrose, lactose, dextrose, sodium chloride, and sorbitol. Lubricants are useful for facilitating tablet manufacture and include vegetable oils, glycerin, magnesium stearate, calcium stearate, and stearic acid. Disintegrants, which are useful for facilitating disintegration of the tablet, generally include starches, clays, celluoses, algins, gums, and crosslinked polymers. Diluents, which are generally included to provide bulk to the tablet, may include dicalcium phosphate, calcium sulfate, lactose, cellulose, kaolin, mannitol, sodium chloride, dry starch, and powdered sugar. Surfactants suitable for use in the formulation according to the present invention may be anionic, cationic, amphoteric, or nonionic surface active agents. Stabilizers may be included in the formulations to inhibit or lessen reactions leading to decomposition of the active agents, such as oxidative reactions.

Solid dosage forms may be formulated so as to provide a delayed release of the active agents, such as by application of a coating. Delayed release coatings are known in the art, and dosage forms containing such may be prepared by any known suitable method. Such methods generally include that, after preparation of the solid dosage form (*e.g*., a tablet or caplet), a delayed release coating composition is applied. Application can be by methods, such as airless spraying, fluidized bed coating, use of a coating pan, or the like. Materials for use as a delayed release coating can be polymeric in nature, such as cellulosic material (*e.g*., cellulose butyrate phthalate, hypromellose phthalate, and carboxymethyl ethylcellulose), and polymers and copolymers of acrylic acid, methacrylic acid, and esters thereof.

Solid dosage forms according to the present invention may also be sustained release (*i.e*., releasing the active agents over a prolonged period of time), and may or may not also be delayed release. Sustained release formulations are known in the are and are generally prepared by dispersing a drug within a matrix of a gradually degradable or hydrolyzable material, such as an insoluble plastic, a hydrophilic polymer, or a fatty compound. Alternatively, a solid dosage form may be coated with such a material.

Formulations for parenteral administration include aqueous and non-aqueous sterile injection solutions, which may further contain additional agents, such as antioxidants, buffers, bacteriostats, and solutes, which render the formulations isotonic with the blood of the intended recipient. The formulations may include aqueous and non-aqueous sterile suspensions, which contain suspending agents and thickening agents. Such formulations for parenteral administration may be presented in unit-dose or multi-dose containers, such as, for example, sealed ampoules and vials, and may be stores in a freeze-dried (lyophilized) condition requiring only the addition of the sterile liquid carrier, for example, water (for injection), immediately prior to use. Extemporaneous injection solutions and suspensions may be prepared from sterile powders, granules, and tablets of the kind previously described.

The combinations according to the present invention may also be administered transdermally, wherein the active agents are incorporated into a laminated structure (generally referred to as a "patch") that is adapted to remain in intimate contact with the epidermis of the recipient for a prolonged period of time. Typically, such patches are available as single layer "drug-in-adhesive" patches or as multi-layer patches where the active agents are contained in a layer separate from the adhesive layer. Both types of patches also generally contain a backing layer and a liner that is removed prior to attachment to the skin of the recipient. Transdermal drug delivery patches may also be comprised of a reservoir underlying the backing layer that is separated from the skin of the recipient by a semi-permeable membrane and adhesive layer. Transdermal drug delivery may occur through passive diffusion or may be facilitated using electrotransport or iontophoresis.

Formulations for rectal delivery of the combinations of the present invention include rectal suppositories, creams, ointments, and liquids. Suppositories may be presented as the active agents in combination with a carrier generally known in the art, such as polyethylene glycol. Such dosage forms may be designed to disintegrate rapidly or over an extended period of time, and the time to complete disintegration can range from a short time, such as about 10 minutes, to an extended period of time, such as about 6 hours.

Topical formulation may be in any form suitable and readily known in the art for delivery of active agents to the body surface, including dermally, buccally, and sublingually. Typical examples of topical formulations include ointments, creams, gels, pastes, and solutions. Formulations for topical administration in the mouth also include lozenges.

The present invention also includes an article of manufacture providing a combination of droxidopa and one or more COMT inhibitor, Cholinesterase inhibitor, MAOI, or combination thereof. The article of manufacture may contain the combinations of compounds described herein in combination with one or more further therapeutic agents. The article of manufacture can include a vial or other container that contains a composition suitable for use according to the present invention together with any carrier, either dried or in liquid form. In particular, the article of manufacture can comprise a kit including a container with a combination according to the invention. In such a kit, the composition can be delivered in a variety of combinations. For example, the composition can comprise a single dosage unit comprising all of the active ingredients (*e.g*., droxidopa and a COMT inhibitor, droxidopa and a Cholinesterase inhibitor, droxidopa and an MAOI, or droxidopa and two or more of a COMT inhibitor, cholinesterase inhibitor, and MAOI). Alternately, the composition can comprise multiple dosage units, each comprising a single active ingredient, the dosages being intended for administration in combination, in succession, or in other close proximity of time. For example, the dosages could be solid forms (*e.g*., tablets, caplets, capsules, or the like) or liquid forms (*e.g*., vials and pre-filled syringes), each comprising a single active ingredient, but being provided in blister packs, bags, or the like, for administration in combination.

The article of manufacture further includes instructions in the form of a label on the container and/or in the form of an insert included in a box in which the container is packaged, for the carrying out the method of the invention. The instructions can also be printed on the box in which the vial is packaged. The instructions contain information such as sufficient dosage and administration information so as to allow the subject or a worker in the field to administer the pharmaceutical composition. It is anticipated that a worker in the field encompasses any doctor, nurse, technician, spouse, or other caregiver that might administer the composition. The pharmaceutical composition can also be self-administered by the subject.

### EXAMPLE

### Pharmacokinetic Properties of Droxidopa Combinations

The present invention will now be described with specific reference to the following example. The following example is not intended to be limiting of the invention and is rather provided as exemplary embodiments.

The pharmacokinetic properties of droxidopa combinations according to the invention were evaluated in male Sprague Dawley rats. Four test groups with four rats in each group were established. Group 1 was administered droxidopa alone as a baseline group. Group 2 was administered droxidopa in combination with the COMT inhibitor entacapone. Group 3 was administered droxidopa in combination with the cholinesterase inhibitor pyridostigmine. Group 4 was administered droxidopa in combination with the MAOI nialamide. For each group, the droxidopa or droxidopa combination was formulated with a vehicle formed of a water solution containing 1% carboxymethylcellulose with 0.2% TWEEN^{®} 80 emulsifier. The weights and of the droxidopa, entacapone, pyridostigmine, nialamide, and the vehicle provided in the various formulations are shown in Table 1. The calculated concentrations for each component are separately provided in Table 2. The amounts of entacapone, pyridostigmine, and nialamide used in formulations 2-7 were provided as "low" doses and "high" doses based upon disclosure in the literature of generally accepted dosage ranges for their respective known indications. Accordingly

**Table 1**

| Formulation | Formulation Components-weight (g). | | | | |
|---|---|---|---|---|---|
| | Vehicle | Droxidopa | Entacapone | Pyridostigmine | Nialamide |
| 1 | 13.87 g | 0.280g | | | |
| 2 | 13.65 g | 0.280g | 0.0084 g | | |
| 3 | 13.60g | 0.280g | 0.0842g | | |
| 4 | 13.53 g | 0.280g | | 0.0028g | |
| 5 | 13.60g | 0.280g | | 0.0563 g | |
| 6 | 13.61 g | 0.280g | | | 0.0028g |
| 7 | 13.70 g | 0.280g | | | 0.0842g |

**Table 2**

| Formulation | Formulation Components - concentration (mg/g) | | | |
|---|---|---|---|---|
| | Droxidopa | Entacapone | Pyridostigmine | Nialamide |
| 1 | 19.81 mg/g | | | |
| 2 | 20.11mg/g | 0.603 mg/g | | |
| 3 | 20.08 mg/g | 6.031 mg/g | | |
| 4 | 20.30 mg/g | | 0.203 mg/g | |
| 5 | 20.20 mg/g | | 4.042 mg/g | |
| 6 | 20.15 mg/g | | | 0.202 mg/g |
| 7 | 20.04 mg/g | | | 5.985 mg/g |

Rats in each group were given a single gavage dose of droxidopa alone or the droxidopa combination, the time of dosing being recorded as time = 0. Dosing was based upon the weight of the subject and was adjusted to provide all test subjects a droxidopa dose of approximately 100 mg per kg of body weight. Blood samples (approximately 100 µL) were collected at approximately 5, 15, and 30 minutes, and at approximately 1, 2, 4, 8, and 24 hours after dosing. Dosing and blood collection were via an indwelling jugular vein cannula. Blood samples were drawn into a heparinized 1 mL syringe (charged with 5 µL of heparin solution [1000 U/mL]) and then transferred to a microcentrifuge.

Acetonitrile (100 µL) containing 0.2% formic acid was added to 25 µL of each plasma sample in a microcentrifuge tube. Internal standard (5 µL of 4 µg/mL 3,4-dihydroxybenzylamine (DHBA) in acetonitrile) was added, and the samples were vortexed and centrifuged to precipitate protein. The supernatant was transferred to an autosample vial with insert and injected on an Applied Biosystems API 4000 Liquid Chromatography-Mass Spectrometer (LC-MS) apparatus interfaced with an Agilent 100 High Pressure Liquid Chromatography (HPLC) apparatus. Data was collected and processed using Analyst software. The autosampler was cooled to 4 °C, and the sample injection volume 5 µL. Chromatography was conducted on a Waters Atlantis dC18 column (25 cm x 4.6 mm, 5 µm) with guard column. The solvent was water containing 0.2% formic acid, and the flow rate was set at 0.8 mL/min.

Plasma droxidopa concentration in the rat test subjects following administration of droxidopa alone or droxidopa combinations according to the invention is provided in Table 3. As a standard, plasma droxidopa concentration in rats administered the drug vehicle with no droxidopa or droxidopa combinations present was also evaluated, and no droxidopa was detected over a 24 hour period in plasma from the rats administered the vehicle alone. Likewise, no droxidopa was detected in any subjects prior to dosing of the droxidopa or droxidopa combinations. As seen in Table 3, plasma droxidopa concentration reached a maximum concentration for all formulations in a time of approximately 1-2 hours following dosing.

**Table 3**

| Formulation | Mean Plasma Dioxidopa Concentration µg/mL) at Time Post-Dosage | | | | | | | |
|---|---|---|---|---|---|---|---|---|
| | 0.083 hr | 0.25 hr | 0.5 hr | 1 hr | 2 hr | 4 hr | 8 hr | 24 hr |
| 1 | 0.401 | 3.996 | 7.869 | 11.336 | 10.548 | 3.391 | 0.610 | 0.001 |
| 2 | 0.328 | 3.245 | 8.641 | 12.050 | 8.772 | 4.795 | 3.054 | 0.005 |
| 3 | 0.189 | 2.799 | 6.775 | 8.440 | 9.270 | 3.425 | 1.853 | 0.010 |
| 4 | 0.459 | 3.570 | 7.941 | 10.054 | 8.650 | 2.976 | 1.795 | 0.005 |
| 5 | 0.456 | 4.033 | 7.341 | 8.380 | 5.989 | 2.429 | 0.431 | 0.002 |
| 6 | 0.493 | 2.867 | 6.807 | 8.579 | 6.065 | 1.829 | 0.297 | 0.000 |
| 7 | 0.311 | 3.017 | 6.506 | 7.886 | 6.381 | 2.380 | 1.535 | 0.113 |

Administration of droxidopa combinations was seen to affect plasma norepinephrine concentration in comparison to administration of droxidopa alone. Mean plasma norepinephrine concentration at 2 hours post-dosing with the various formulations tested is provided in Table 4. Formulation 0 indicates administration of the vehicle alone without droxidopa or a droxidopa combination of the invention and provides a baseline comparative of plasma norepinephrine levels in an untreated subject.

**Table 4**

| Formulation | Plasma Norepinephrine Concentration (pg/µL) |
|---|---|
| 0 | 0.711 |
| 1 | 3.320 |
| 2 | 3.358 |
| 3 | 6.359 |
| 4 | 4.000 |
| 5 | 2.290 |
| 6 | 2.182 |
| 7 | 2.674 |

As seen in Table 4, administration of droxidopa alone caused an approximate 5-fold increase in plasma norepinephrine concentration. Treatment with droxidopa in combination with the COMT inhibiting compound caused an even greater increase in plasma norepinephrine concentration. Treatment with droxidopa in combination with a relatively low dose of the cholinesterase inhibiting compound similarly caused an increase in plasma norepinephrine concentration in relation to treatment with droxidopa alone; however, the plasma norepinephrine concentration was reduced in relation to treatment with droxidopa alone when a combination of droxidopa with a relatively high dose of the cholinesterase inhibiting compound was used. Plasma norepinephrine concentration after treatment with both combinations of droxidopa with the MAOI compound was reduced relative to treatment with droxidopa alone.

Mean values for various pharmacokinetic properties of the inventive combinations used in the above study are provided in Table 5. Specifically, Table 5 provides the terminal elimination half-life (T_{1/2}) of the administered formulations, the maximum observed concentration (Cₘₐₓ) of the active ingredients in each formulation, the time to reach the maximum observed concentration (Tₘₐₓ), the area under the plasma concentration-time curve from time zero to the last measured time point (AUCₐₗₗ), and the observed volume of distribution at steady state (Vz_F_obs). Note that for extravascular models, the fraction of dose absorbed cannot be estimated. Therefore, Vz_F_obs for such models is actually Volume/F where F is the fraction of dose absorbed.

**Table 5**

| Formulation | T_{1/2} (hr) | Cₘₐₓ (µg/mL) | Tₘₐₓ (hr) | AUCₐₗₗ (hr µg/mL) | Vz_F_obs (mL/kg) |
|---|---|---|---|---|---|
| 1 | 1.4 | 11.4 | 1.25 | 44.4 | 5270.4 |
| 2 | 1.86 | 12.4 | 1.125 | 71.1 | 5016.4 |
| 3 | 2.64 | 10.2 | 1.38 | 52.3 | 8854.1 |
| 4 | 1.93 | 10.1 | 1.25 | 51.2 | 5900.8 |
| 5 | 1.79 | 8.4 | 1 | 30.5 | 8763.6 |
| 6 | 1.41 | 8.6 | 1 | 27.2 | 8030.0 |
| 7 | 3.77 | 8.1 | 0.875 | 42.0 | 16404.1 |

As seen above, the invention is useful for increasing droxidopa half-life, and such increase can be seen in a variety of pathways, such as through an effect on drug metabolism, volume of distribution of the drug, or a combination of the two. For example, the increase in half-life arising from the combination with entacapone indicates peripheral activity to block the metabolism of droxidopa to 3-OM-droxidopa (the major metabolite of droxidopa), thus increasing residence time of droxidopa in the body. Similarly, an increase in the volume of distribution indicates a decrease in the amount of drug available to organs of elimination, which can further affect half-life. The increase in half-life associated with the relatively high dose of nialamide is surprising since MAOIs are not typically considered to be a major metabolic pathway for droxidopa, and is likely the result of the unexpected increase in the apparent volume of distribution. Similarly, the combination with pyridostigmine also surprisingly led to an increase in droxidopa half-life even though cholinesterase compounds would generally not be expected to affect droxidopa metabolism. Droxidopa half-life when administered alone or in combination with entacapone, pyridostigmine, or nialamide is graphically illustrated in FIG. 1.

Many modifications and other embodiments of the inventions set forth herein will come to mind to one skilled in the art to which these inventions pertain having the benefit of the teachings presented in the foregoing descriptions. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. Use of droxidopa in combination with one or more additional compounds selected from the group consisting of catechol-O-methyltransferase inhibiting compounds, Cholinesterase inhibiting compounds, monoamine oxidase inhibiting compounds, and combinations thereof as a composition for increasing the half-life of the droxidopa in a mammal when administered to the mammal for treating a condition responsive to droxidopa.

2. The use of claim 1, wherein the half-life of the droxidopa is increased by at least 20%.

3. The use of claim 1, wherein the half-life of the droxidopa is increased by at least 50%.

4. The use of claim 1, wherein the droxidopa and the one or more additional compounds are administered as a single dosage unit.

5. The use of claim 1, wherein the droxidopa and the one or more additional compounds are administered simultaneously or sequentially.

6. The use of claim 1, wherein the one or more additional compounds is a catechol-O-methyltransferase inhibiting compound selected from the group consisting of entacapone, tolcapone, nitecapone, and combinations thereof.

7. The use of claim 1, wherein the one or more additional compounds is a cholinesterase inhibiting compound selected from the group consisting of pyridostigmine, donepezil, rivastigmine, galantamine, tacrine, neostigmine, metrifonate, physostigmine, ambenonium, demarcarium, thiaphysovenine, phenscrine, edrophonium, cymserine and combinations thereof.

8. The use of claim 1, wherein the one or more additional compounds is a monoamine oxidase inhibiting compound selected from the group consisting of isocarboxazid, moclobemide, phenelzine, tranylcypromine, selegiline, nialamide., iproniazid, iproclozide, toloxatone, harmala, brofaromine, benmoxin, 5-methoxy-N,N-dimetbyltryptamine, 5-methoxy-α-methyltryptamine, and combinations thereof.

9. The use of claim 1, wherein the one or more additional compounds is a monoamine oxidase inhibiting compound that is selective for the MAO-B isoform.

10. The use of claim 1, wherein the one or more additional compounds is a monoamine oxidase inhibiting compound that is selective for the MAO-A isoform.

11. The use of claim 1, wherein the one or more additional compounds comprise entacapone.

12. The use of claim 1, wherein the one or more additional compounds comprise pyridostigmine.

13. The use of claim 1, wherein the one or more additional compounds comprise donepezil.

14. The use of claim 1, wherein the one or more additional compounds comprise nialamide.

15. The use of claim 1, wherein the increased half-life of the droxidopa allows for the composition to have a reduced dosage of the droxidopa without limiting effective treatment of the condition responsive to droxidopa when the composition is administered to the mammal.

16. The use of claim 1, wherein the increased half-life of the droxidopa allows for the composition to be administered such that there is a reduced dosing frequency of the droxidopa without limiting effective treatment of the condition responsive to droxidopa when the composition is administered to the mammal.

17. Use of droxidopa in combination with one or more additional compounds selected from the group consisting of catechol-O-methyltransferase inhibiting compounds, cholinesterase inhibiting compounds, monoamine oxidase inhibiting compounds, and combinations thereof in the manufacture of a composition for increasing the volume of distribution of the droxidopa in a mammal when administered to the mammal for treating a condition responsive to droxidopa.

18. The use of claim 17, wherein the volume of distribution of the droxidopa is increased by at least 20%.

19. The use of claim 17, wherein the volume of distribution of the droxidopa is increased by at least 50%.

20. The use of claim 17, wherein the droxidopa and the one or more additional compounds are administered in the same dosage unit.

21. The use of claim 17, wherein the droxidopa and the one or more additional compounds are administered simultaneously or sequentially.

22. The use of claim 17, wherein the one or more additional compounds is a catechol-O-methyltransferase inhibiting compound selected from the group consisting of entacapone, tolcapone, nitecapone, and combinations thereof.

23. The use of claim 17, wherein the one or more additional compounds is a cholinesterase inhibiting compound selected from the group consisting of pyridostigmine, donepezil, rivastigmine, galantamine, tacrine, neostigmine, metrifonate, physostigmine, ambenonium, demarcarium, thiaphysovenine, phenserine, edrophonium, cymserine and combinations thereof

24. The use of claim 17, wherein the one or more additional compounds is a monoamine oxidase inhibiting compound selected from the group consisting of isocarboxazid, moclobemide, phenelzine, tranylcypromine, selegiline, nialamide, iproniazid, iproclozide, toloxatone, harmala, brofaromine, benmoxin, 5-methoxy-N,N-dimethyltryptamine, 5-methoxy-α-mcthyltryptamine, and combinations thereof.

25. The use of claim 17, wherein the one or more additional compounds is a monoamine oxidase inhibiting compound that is selective for the MAO-B isoform.

26. The use of claim 17, wherein the one or more additional compounds is a monoamine oxidase inhibiting compound that is selective for the MAO-A isoform.

27. The use of claim 17, wherein the one or more additional compounds comprise entacapone.

28. The use of claim 17, wherein the one or more additional compounds comprise pyridostigmine.

29. The use of claim 17, wherein the one or more additional compounds comprise donepezil.

30. The use of claim 17, wherein the one or more additional compounds comprise nialamide.
